# EUROPEAN PATENT APPLICATION

(11) **EP 4 083 189 A1**
(43) Date of publication of application: **02.11.2022**
(21) Application number: 22170751.6
(22) Date of filing: 29.04.2022
(51) Int. Cl.: C12N 1/20, A61K 8/98

(54) **NEW ISOLATED STRAIN OF LACTOBACILLUS AND USES THEREOF**

(30) Priority: 30.04.2021 IT 202100011006
(71) Applicant: Institut Agricole Regional, 11100 Aosta (IT); Regione Autonoma Valle d'Aosta, 11100 Aosta (IT)
(72) Inventor: FLUTTO, Tania, I-11018 VILLENEUVE (Aosta) (IT); VALENTINI, Sabina, I-11010 SARRE (Aosta) (IT); GUGLIELMO, Fabio, I-11020 FENIS (Aosta) (IT); BRUNET, Ilaria, I-11017 MORGEX (Aosta) (IT)
(74) Representative: Rimini, Rebecca

(57) **Abstract**

The invention relates to a new isolated strain of *Lactobacillus delbruekii* and its use in a method for preparing a dermatological composition for topical application to the skin. The dermatological composition obtainable through the method of the invention, which is particularly effective in promoting skin repair processes, is also described herein.

## Description

The present invention relates to a new strain of *Lactobacillus delbrueckii* and its use for preparing a dermatological composition.

As a mediator between the body and the outside world, the skin is an excellent protective barrier against several harmful substances, while preventing uncontrolled loss of water and solutes.

The skin is normally populated by millions of microorganisms, including, for example, bacteria, fungi, and viruses, which make up the microbiota. As happens in the intestine, these symbiotic microorganisms occupy several niches and exert a beneficial action against the invasion of many pathogenic species. In addition, the skin microbiota also plays a major role in training the immune system, particularly the T cells on the skin, by inducing its response against the invasion of pathogenic organisms.

Conditions of disruption of the skin barrier or the presence of an imbalance between commensal and pathogenic microorganisms result in the onset of skin diseases and, in some cases, even in the onset of systemic diseases.

In this context, therefore, there is a need to provide dermatological treatments which are effective in preserving the integrity and functionality of the skin barrier, thereby allowing their effective, safe, and simple use in the clinical and/or cosmetological field.

These and other needs are met by the present invention which provides a new isolated strain of *Lactobacillus delbrueckii,* a method for preparing a dermatological composition using said lactobacillus, and the composition obtainable through said method, as defined in the appended independent claims.

Further features and advantages of the invention are identified in the appended dependent claims and illustrated in detail in the following description.

The appended independent and dependent claims form an integral part of the present specification.

As will be illustrated in greater detail in the following experimental part, the present inventors have isolated for the first time a new Valle d'Aosta native bacterial strain, designated herein as MF-20/7A/24, and defined it as belonging to the *Lactobacillus delbrueckii* species by amplifying and sequencing a portion of the genomic region encoding for bacterial 16S ribosomal RNA (16SrDNA).

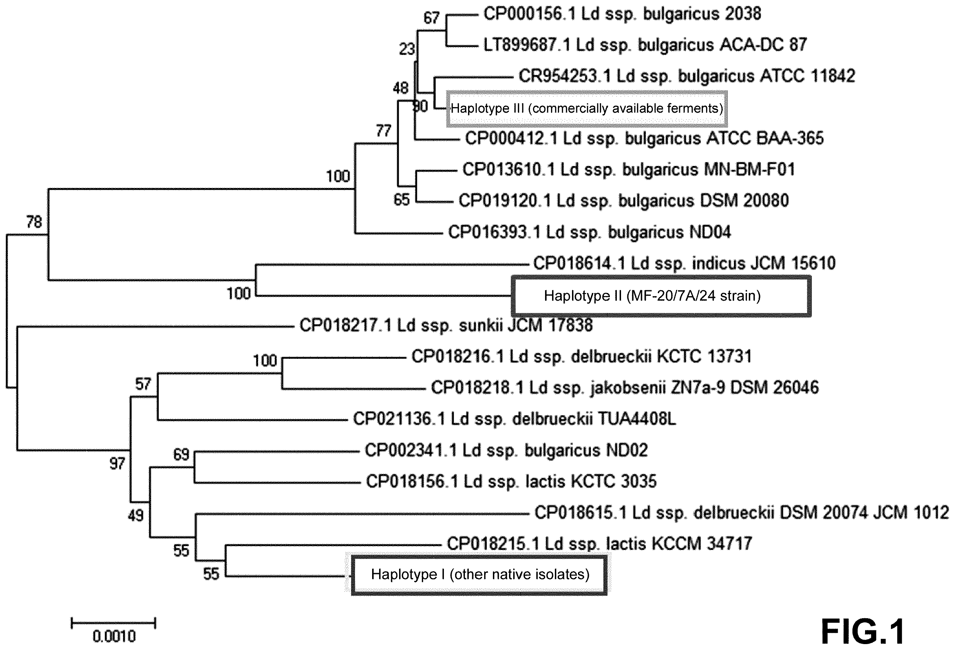

Furthermore, the phylogenetic analysis based on the sequencing of eight additional bacterial genes as shown in Figure 1 revealed the uniqueness of haplotype II corresponding to the MF-20/7A/24 isolate compared to other native Valle d'Aosta lactobacilli (haplotype I) and commercially available lactobacilli (haplotype III), as well as its phylogenetic proximity to the subspecies indicus of *Lactobacillus delbrueckii.* A comparative transcriptomics study conducted by the present inventors comparing the *Lactobacillus* object of the present invention with a commercially available *Lactobacillus delbrueckii* strain then revealed that genes involved in the metabolism of galactose and butyric acid are surprisingly active in the *Lactobacillus* of the invention. Subsequent microbiological analyses confirmed that, unlike other *Lactobacilli delbrueckii,* the *Lactobacillus* object of the present invention surprisingly possesses the natural ability to catabolize galactose through the Leloir metabolic pathway, which so far has only been described in *Lactobacillus helveticus* and *Lactococcus lactis* (Iskandar C.F., et al., "Review of lactose and galactose metabolism in Lactic Acid Bacteria dedicated to expert genomic annotation". Trends in Food Science & Technology, 88 (2019), 121-132; Grossiord B., et al. "Genetic galactose utilization via the Leloir pathway in lactic acid bacteria". Lait (1998), 78, 77-84).

It is therefore an object of the present invention to provide an isolated strain of *Lactobacillus delbrueckii,* characterised in that it comprises:
(a) a genomic nucleic acid sequence encoding 16S ribosomal RNA comprising the nucleotide sequence of SEQ ID NO. 1; and
(b) a genomic nucleic acid sequence selected from the group consisting of a sequence encoding the ATP-dependent Clp protease ATP-binding subunit ClpX (ClpX) comprising the nucleotide sequence of SEQ ID NO. 2, a sequence encoding the chromosomal replication initiation protein DnaA comprising the nucleotide sequence of SEQ ID NO. 3, a sequence encoding the GroEL protein comprising the nucleotide sequence of SEQ ID NO. 4, a sequence encoding the UDP-N-acetylmuramoyl-L-alanyl-D-glutamate-L-lysine ligase protein (MurE) comprising the nucleotide sequence of SEQ ID NO. 5, a sequence encoding the phenylalanine-tRNA ligase alpha subunit (PheS) comprising the nucleotide sequence of SEQ ID NO. 6, a sequence encoding the CTP synthetase (PyrG) comprising the nucleotide sequence of SEQ ID NO. 7, a sequence encoding recombinase A (RecA) comprising the nucleotide sequence of SEQ ID NO. 8, a sequence encoding the DNA-dependent RNA polymerase beta subunit (RpoB) comprising the nucleotide sequence of SEQ ID NO. 9, a sequence encoding the beta-galactosidase protein comprising the nucleotide sequence of SEQ ID NO. 28, a sequence encoding the galactose-1-phosphate uridylyltransferase protein (GalT) comprising the nucleotide sequence of SEQ ID NO. 29, a sequence encoding the galactokinase protein comprising the nucleotide sequence of SEQ ID NO. 30, a sequence encoding the UDP-galactopyranose mutase protein (GIF) comprising the nucleotide sequence of SEQ ID NO. 31, a sequence encoding the UDP-glucose 4-epimerase protein (GalE) comprising the nucleotide sequence of SEQ ID NO. 32, a sequence encoding the pyruvate kinase protein comprising the nucleotide sequence of SEQ ID NO. 33, a sequence encoding the phosphoenolpyruvate carboxylase protein comprising the nucleotide sequence of SEQ ID NO. 34, a sequence encoding the pyruvate oxidase protein (Pox5_1) comprising the nucleotide sequence of SEQ ID NO. 35, a sequence encoding the pyruvate oxidase protein (Pox5_2) comprising the nucleotide sequence of SEQ ID NO. 36, a sequence encoding the acetate kinase protein comprising the nucleotide sequence of SEQ ID NO. 37, a sequence encoding the succinate-semialdehyde dehydrogenase protein [NADP(+)] (GabD) comprising the nucleotide sequence of SEQ ID NO. 38, a sequence encoding the acetyl-CoA acetyltransferase protein comprising the nucleotide sequence of SEQ ID NO. 39, and any combination thereof.

In one embodiment, the isolated strain of *Lactobacillus delbrueckii* object of the invention comprises a genomic nucleic acid sequence encoding 16S ribosomal RNA comprising the nucleotide sequence of SEQ ID NO. 1, a genomic nucleic acid sequence encoding the galactose-1-phosphate uridylyltransferase protein (GalT) comprising the nucleotide sequence of SEQ ID NO. 29, a genomic nucleic acid sequence encoding the succinate-semialdehyde dehydrogenase protein [NADP(+)] (GabD) comprising the nucleotide sequence of SEQ ID NO. 38, and a genomic nucleic acid sequence encoding the acetyl-CoA acetyltransferase protein comprising the nucleotide sequence of SEQ ID NO. 39.

In another embodiment, the isolated strain of *Lactobacillus delbrueckii* object of the invention comprises a genomic nucleic acid sequence encoding 16S ribosomal RNA comprising the nucleotide sequence of SEQ ID NO. 1, a genomic nucleic acid sequence encoding the beta-galactosidase protein comprising the nucleotide sequence of SEQ ID NO. 28, a genomic nucleic acid sequence encoding the galactose-1-phosphate uridylyltransferase protein (GalT) comprising the nucleotide sequence of SEQ ID NO. 29, a genomic nucleic acid sequence encoding the galactokinase protein comprising the nucleotide sequence of SEQ ID NO. 30, a genomic nucleic acid sequence encoding the UDP-galactopyranose mutase protein (GIF) comprising the nucleotide sequence of SEQ ID NO. 31, and a genomic nucleic acid sequence encoding the UDP-glucose 4-epimerase protein (GalE) comprising the nucleotide sequence of SEQ ID NO. 32.

In yet another embodiment, the isolated strain of *Lactobacillus delbrueckii* object of the invention comprises a genomic nucleic acid sequence encoding 16S ribosomal RNA comprising the nucleotide sequence of SEQ ID NO. 1, a genomic nucleic acid sequence encoding the pyruvate kinase protein comprising the nucleotide sequence of SEQ ID NO. 33, a genomic nucleic acid sequence encoding the phosphoenolpyruvate carboxylase protein comprising the nucleotide sequence of SEQ ID NO. 34, a genomic nucleic acid sequence encoding the pyruvate oxidase protein (Pox5_1) comprising the nucleotide sequence of SEQ ID NO. 35, a genomic nucleic acid sequence encoding the pyruvate oxidase protein (Pox5_2) comprising the nucleotide sequence of SEQ ID NO. 36, a genomic nucleic acid sequence encoding the acetate kinase protein comprising the nucleotide sequence of SEQ ID NO. 37, a genomic nucleic acid sequence encoding the succinate-semialdehyde dehydrogenase protein [NADP(+)] (GabD) comprising the nucleotide sequence of SEQ ID NO. 38, and a genomic nucleic acid sequence encoding the acetyl-CoA acetyltransferase protein comprising the nucleotide sequence of SEQ ID NO. 39.

Still in another embodiment, the isolated strain of *Lactobacillus delbrueckii* object of the invention comprises a genomic nucleic acid sequence encoding 16S ribosomal RNA comprising the nucleotide sequence of SEQ ID NO. 1, a genomic nucleic acid sequence encoding the beta-galactosidase protein comprising the nucleotide sequence of SEQ ID NO. 28, a genomic nucleic acid sequence encoding the galactose-1-phosphate uridylyltransferase protein (GalT) comprising the nucleotide sequence of SEQ ID NO. 29, a genomic nucleic acid sequence encoding the galactokinase protein comprising the nucleotide sequence of SEQ ID NO. 30, a genomic nucleic acid sequence encoding the UDP-galactopyranose mutase protein (GIF) comprising the nucleotide sequence of SEQ ID NO. 31, a genomic nucleic acid sequence encoding the UDP-glucose 4-epimerase protein (GalE) comprising the nucleotide sequence of SEQ ID NO. 32, a genomic nucleic acid sequence encoding the pyruvate kinase protein comprising the nucleotide sequence of SEQ ID NO. 33, a genomic nucleic acid sequence encoding the phosphoenolpyruvate carboxylase protein comprising the nucleotide sequence of SEQ ID NO. 34, a genomic nucleic acid sequence encoding the pyruvate oxidase protein (Pox5_1) comprising the nucleotide sequence of SEQ ID NO. 35, a genomic nucleic acid sequence encoding the pyruvate oxidase protein (Pox5_2) comprising the nucleotide sequence of SEQ ID NO. 36, a genomic nucleic acid sequence encoding the acetate kinase protein comprising the nucleotide sequence of SEQ ID NO. 37, a genomic nucleic acid sequence encoding the succinate-semialdehyde dehydrogenase protein [NADP(+)] (GabD) comprising the nucleotide sequence of SEQ ID NO. 38, and a genomic nucleic acid sequence encoding the acetyl-CoA acetyltransferase protein comprising the nucleotide sequence of SEQ ID NO. 39.

According to a preferred embodiment, the isolated strain of *Lactobacillus delbrueckii* object of the invention comprises a genomic nucleic acid sequence encoding 16S ribosomal RNA comprising the nucleotide sequence of SEQ ID NO. 1, a genomic nucleic acid sequence encoding the ATP-dependent Clp protease ATP-binding subunit ClpX (ClpX) comprising the nucleotide sequence of SEQ ID NO. 2, a genomic nucleic acid sequence encoding the chromosomal replication initiation protein DnaA comprising the nucleotide sequence of SEQ ID NO. 3, a genomic nucleic acid sequence encoding the GroEL protein comprising the nucleotide sequence of SEQ ID NO. 4, a genomic nucleic acid sequence encoding the UDP-N-acetylmuramoyl-L-alanyl-D-glutamate-L-lysine ligase protein (MurE) comprising the nucleotide sequence of SEQ ID NO. 5, a genomic nucleic acid sequence encoding the phenylalanine-tRNA ligase alpha subunit (PheS) comprising the nucleotide sequence of SEQ ID NO. 6, a genomic nucleic acid sequence encoding the CTP synthetase (PyrG) comprising the nucleotide sequence of SEQ ID NO. 7, a genomic nucleic acid sequence encoding recombinase A (RecA) comprising the nucleotide sequence of SEQ ID NO. 8, a genomic nucleic acid sequence encoding the DNA-dependent RNA polymerase beta subunit (RpoB) comprising the nucleotide sequence of SEQ ID NO. 9, a genomic nucleic acid sequence encoding the beta-galactosidase protein comprising the nucleotide sequence of SEQ ID NO. 28, a genomic nucleic acid sequence encoding the galactose-1-phosphate uridylyltransferase protein (GalT) comprising the nucleotide sequence of SEQ ID NO. 29, a genomic nucleic acid sequence encoding the galactokinase protein comprising the nucleotide sequence of SEQ ID NO. 30, a genomic nucleic acid sequence encoding the UDP-galactopyranose mutase protein (GIF) comprising the nucleotide sequence of SEQ ID NO. 31, a genomic nucleic acid sequence encoding the UDP-glucose 4-epimerase protein (GalE) comprising the nucleotide sequence of SEQ ID NO. 32, a genomic nucleic acid sequence encoding the pyruvate kinase protein comprising the nucleotide sequence of SEQ ID NO. 33, a genomic nucleic acid sequence encoding the phosphoenolpyruvate carboxylase protein comprising the nucleotide sequence of SEQ ID NO. 34, a genomic nucleic acid sequence encoding the pyruvate oxidase protein (Pox5_1) comprising the nucleotide sequence of SEQ ID NO. 35, a genomic nucleic acid sequence encoding the pyruvate oxidase protein (Pox5_2) comprising the nucleotide sequence of SEQ ID NO. 36, a genomic nucleic acid sequence encoding the acetate kinase protein comprising the nucleotide sequence of SEQ ID NO. 37, a genomic nucleic acid sequence encoding the succinate-semialdehyde dehydrogenase protein [NADP(+)] (GabD) comprising the nucleotide sequence of SEQ ID NO. 38, and a genomic nucleic acid sequence encoding the acetyl-CoA acetyltransferase protein comprising the nucleotide sequence of SEQ ID NO. 39.

Under the Budapest Treaty on the International Recognition of the Deposit of Microorganisms for the Purposes of Patent Procedure, the new *Lactobacillus delbrueckii* strain isolated by the present inventors was deposited on March 12, 2020, at the Belgian Coordinated Collections of Microorganisms (BCCM) Laboratorium voor Microbiologie - Bacteriënverzameling (LMG) International Depository Authority, Universiteit Gent, K.L. Ledeganckstraat 35, 9000 Gent, Belgium, under accession number LMG P-31789.

Microbiological and physiological characteristics of the isolated *Lactobacillus delbrueckii* strain which forms the object of the present invention are illustrated in Table 1 below.

**Table 1**

| | |
|---|---|
| Morphology | Bacillus |
| Growth at 30°C. | positive |
| Growth at 37°C. | positive |
| Growth at 40°C. | positive |
| Growth at 42°C. | positive |
| Growth medium | MRS under anaerobic conditions |
| M.I.C. (minimum inhibitory concentration) for vancomycin | >256 µg/ml |
| Catalase | negative |
| Lactose fermentation | positive |
| Galactose fermentation | positive |
| Glucose fermentation | positive |
| ONPG (presence of the β-galactosidase enzyme) | positive |

The microbiological analysis methods are well established and the selection of the most appropriate techniques to be used within the scope of the present invention is well within the skills of those of ordinary skill in the art.

Surprisingly, the present inventors have found that the use of the above-mentioned lactobacillus strain in a method for processing dairy whey, in particular sweet whey, makes it possible to obtain a dermatological composition endowed with beneficial activities on the skin, capable of restoring and maintaining the skin barrier as well as accelerating skin repair processes.

Therefore, also within the scope of the invention is a method for preparing a dermatological composition for topical application to the skin, employing sweet dairy whey as the starting material, the method comprising the steps of:
(i) removing the lipid and protein components from said whey, thereby obtaining an aqueous fraction of the sweet dairy whey; and
(ii) subjecting said aqueous fraction to fermentation by using the isolated strain of *Lactobacillus delbruekii* as previously defined,
thereby obtaining the dermatological composition for topical application to the skin.

The term "sweet dairy whey", as used herein, is intended to mean a liquid by-product from the production of soft, semi-hard or hard cheese, which results from the enzymatic coagulation of casein and has a pH value of between 6 and 7.

Typically, sweet dairy whey has a yellowish colour and is composed of 93-94% water, with a dry matter equal to about 6-7%, whose constituents are substantially: lactose (70%), protein (10%), mineral salts (15% including NaCl, KCl, and mainly calcium phosphates) and other constituents such as lactate and citrate, fats, nitrogenous compounds (urea and uric acid), and B-group vitamins.

Bioactive compounds commonly present in sweet dairy whey are galacto-oligosaccharides (GOS), non-digestible carbohydrates consisting of galactose units, known for their property of favouring the growth of microbial flora, particularly the gut flora, (Torres D.P.M. et al, Compr. Rev. Food Sci. Food Saf. 2010, 9, 438-454; Hingu M.N. and Shah H.S. The Microbes 2013. Vol: 5, ISSN: 2321-3728 (Online)) as well as butyric acid. This fatty acid is an important energy source for eukaryotic cell systems, is a known modulator of transmembrane receptors, and its supplementation results in an improvement in the mitochondrial function (Mollica M.P., et al, Diabetes. 2017, 66: 1405-1418; McNabney S.M. and Henagan T.M., Nutrients, 2017; 9(12): 1348).

As is known in the art, during the production of cheese, the expulsion of the whey (syneresis) from the curd takes place at 36°C, following enzymatic coagulation and precipitation of the caseins, induced by the addition of calf rennet, containing the chymosin enzyme. This enzyme is capable of hydrolysing the negatively charged end of k-casein, at the peptide bond between the amino acid phenylalanine at position 105 and the amino acid methionine at position 106, leading to the formation of para-k-casein and the release of the glycomacropeptide (GMP) or caseinomacropeptide (CMP) into the whey. Hydrolysed k-casein, located on the surface of the micelles, induces the clustering thereof through interaction with the free minerals in the milk.

In one embodiment of the method according to the invention, the removal of the lipid component from the sweet dairy whey used as the starting material is carried out by thermocalcic precipitation.

Within the scope of the present description, the term "thermocalcic precipitation" refers to a method of thermal treatment of a dairy whey added with calcium ions and in the presence of a pH increase, for example by the addition of sodium hydroxide. Typically, the dairy whey is heated to a temperature of between 50° and 55°C.

In the embodiment described above, the thermocalcic precipitation is preferably followed by a microfiltration step in order to remove the precipitated lipid clusters from the sweet dairy whey.

Preferably, in the method according to the invention, the protein component of the sweet dairy whey is removed by ultrafiltration through a membrane, more preferably by tangential flow ultrafiltration.

The selection of the material and of the most appropriate geometry of the ultrafiltration membrane for use within the scope of the present invention, as well as of the operating parameters of the method, is well within the skills of those of ordinary skill in the art.

As previously indicated, the method according to the invention is characterised in that it uses the *Lactobacillus delbrueckii* strain isolated for the first time by the present inventors, designated as MF-20/7A/24. Said lactobacillus strain is used in order to carry out the step of fermentation of the aqueous fraction of the sweet dairy whey, which is obtained following the removal of the whey lipid and protein components.

The cells of the MF-20/7A/24 strain used in the method according to the invention can be viable or non-replicating, for example inactivated, for example by heat treatment. Alternatively, the cells of the MF-20/7A/24 strain can be used in the freeze-dried form, preferably under conditions which preserve cell viability. Freeze-drying techniques are well-established and known per se to those of ordinary skill in the art.

According to the invention, the fermentation step is preferably carried out at a temperature of 37°C, and/or for a time interval preferably comprised between 22 hours and 26 hours, more preferably for 24 hours.

The composition of the sweet dairy whey varies according to the animal species of origin, such as cow, sheep, goat, or buffalo, the feeding and lactation of the animal.

Preferably, but not by way of limitation, the sweet dairy whey used as the starting material in the method according to the invention is obtained from bovine milk, more preferably from native Valle d'Aosta bovine milk, for example the Pezzata Rossa, Pezzata Nera and/or Castana Valle d'Aosta bovine breed.

Despite its nutritional properties, sweet dairy whey is still a waste product of the dairy chain, and its disposal requires complex procedures.

More specifically, with a biochemical oxygen demand of between 30 and 60 g/l (BOD, a parameter used to estimate the pollutant load through an indirect measurement of the quantity of O₂ necessary for the biological oxidation of the organic matter content present in an aqueous solution, in 5 days), dairy whey contributes significantly to pollution. The biodegradability index of said compound was estimated to be between 0.4 and 0.8, attributable to the lactose and protein content. Therefore, considering that the production of 1 kg of cheese requires 10 kg of milk, from which 9 kg of whey are derived, it can be said that the dairy industry is one of the main sources of production of industrial wastewater in Europe.

In the light of the above, the method according to the invention therefore has the undeniable advantage of allowing reuse of the sweet dairy whey as a raw material for the production of a dermatological composition, in particular a dermatological composition which is highly functional for the well-being of the skin.

As will be illustrated in detail in the following experimental examples, the method according to the invention allows preservation, in the dermatological composition thus produced, of the bioactive components originally present in the sweet dairy whey, in particular the galacto-oligosaccharide component and butyric acid. Importantly, the studies conducted by the present inventors have even revealed a significant increase of this organic acid in the dermatological composition obtained with the method according to the invention compared to the aqueous fraction of the sweet dairy whey depleted of the lipid and protein components.

Further studies carried out by the present inventors have also shown that the composition obtained with the method according to the invention is surprisingly endowed with high regenerating power on the skin cells and high rebalancing power on the skin protective barrier, with no cytotoxic effect, thus exerting a considerable wound healing, repairing, and soothing action.

Therefore, a further object of the present invention is a dermatological composition for topical application to the skin comprising one or more galacto-oligosaccharides and butyric acid, characterised in that it is obtainable through the method as defined above.

According to preferred embodiments, the dermatological composition of the present invention also contains one or more of the following additional components: thickening agents, wetting agents, solubilizing agents, chelating agents, pH adjusting agents, and preservative agents.

The dermatological composition according to the invention was found to be suitable for topical application to the skin. Preferred pharmaceutical dosage forms for this purpose are creams, ointments, gels, emulsions, patches, and gauze.

In view of the advantageous properties mentioned above, the dermatological composition according to the invention is particularly suitable for use in the clinical field. Skin diseases include, for example, but are not limited to, acne, atopic dermatitis, wounds, burns, and ageing skin damage.

Alternatively, the dermatological composition according to the invention finds application in the purely cosmetic field, in order to modify or solve unsightly skin appearance caused solely by physiological processes in subjects not affected by dermatological disease, for example in subjects not affected by acne, atopic dermatitis, wounds, burns, and ageing skin damage.

The invention is further described in the examples below, with reference to the accompanying drawings, wherein:
Figure 1 shows the dendrogram of *L. delbrueckii* strains based on a distance matrix obtained by analysing linked sequences of nine bacterial loci and using the neighbour-joining method. The bootstrap test (1,000 replicas) is shown next to each branch. The evolutionary distances were calculated using the Kimura 2-parameter method and have the number of base substitutions per site as the unit. Haplotype II corresponds to the MF-20/7A/24 isolate; haplotype I is common to the remaining native Valle d'Aosta strains; haplotype III corresponds to commercially available lactobacilli;

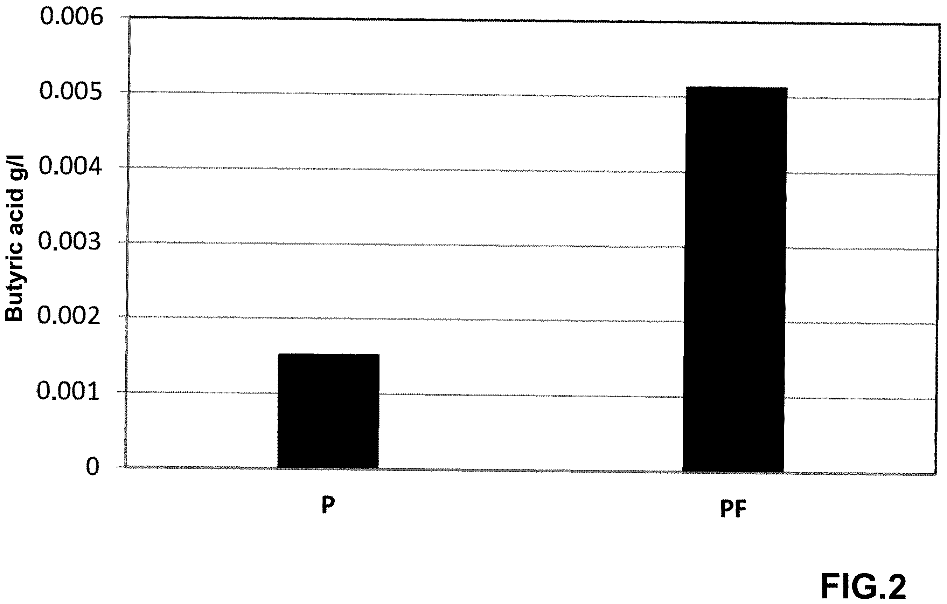

Figure 2 shows butyric acid levels in the aqueous whey fraction after ultrafiltration (permeate, P) and after fermentation with the microorganism of the invention (fermented permeate, FP)

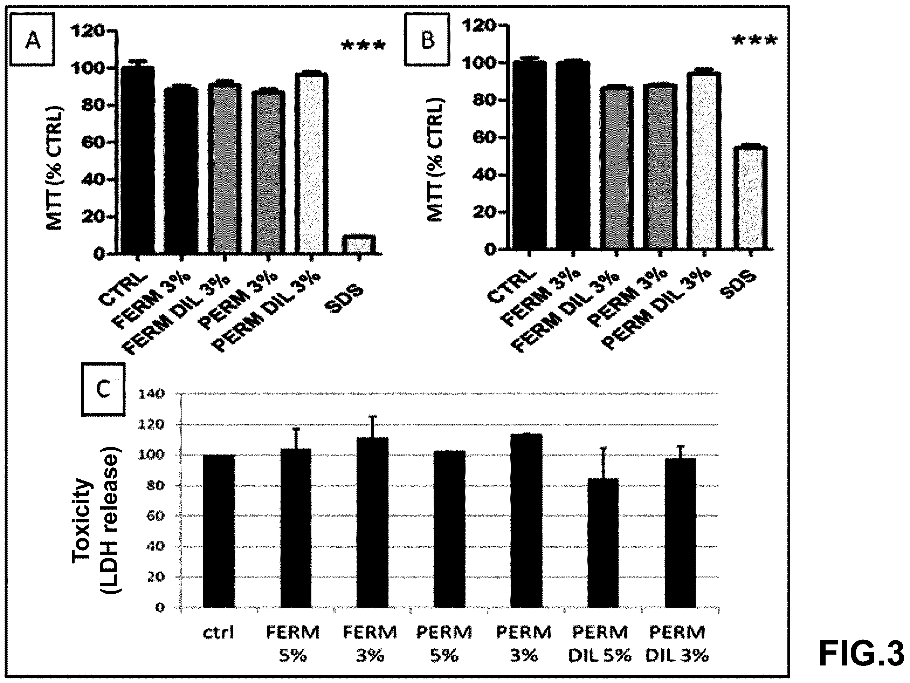

Figure 3 shows graphs depicting the absence of toxicity of the composition of the invention (FERM), at 3% (FERM 3%) and 5% (FERM 5%) concentrations in the culture medium, and 3% dilution (FERM DIL 3%) in the culture medium, determined with the MTT (A), neutral red uptake (NRU) (B) and LDH release (C) assays. The graphs also show the toxicity values of the aqueous whey fraction after thermocalcic precipitation and ultrafiltration (indicated hereinafter as the "aqueous whey fraction"; PERM) at 3% (PERM 3%) and 5% (PERM 5%) concentrations in the culture medium, and 3% (PERM DIL 3%) and 5% (PERM DIL 5%) dilutions in the culture medium. In the MTT and NRU assays, the sodium dodecyl sulfate (SDS) compound was used as a positive control for toxicity;

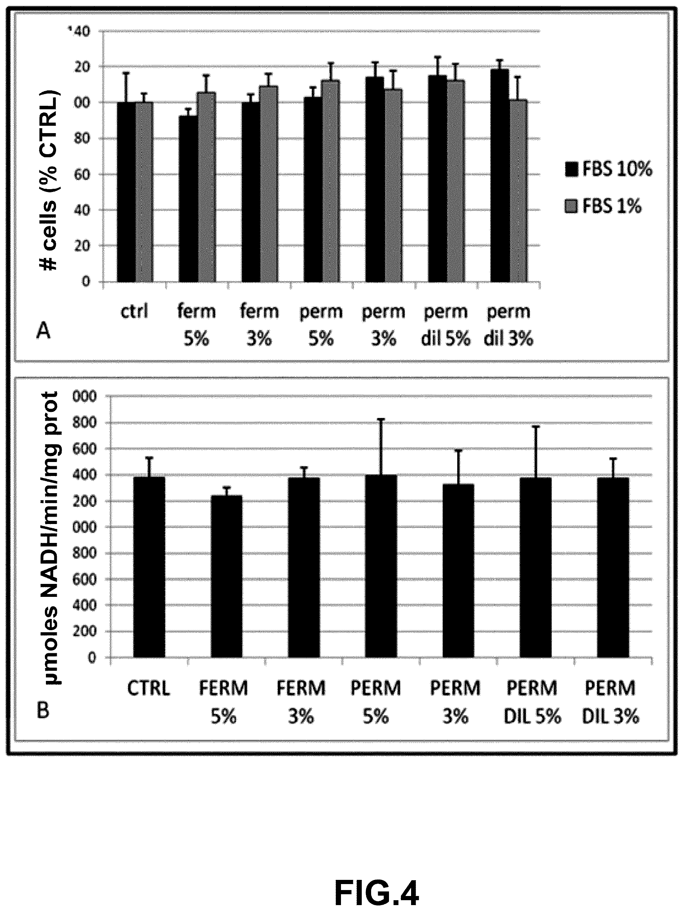

Figure 4 shows graphs depicting the absence of cell proliferation stimulus of the composition of the invention (FERM) and of the "aqueous whey fraction" (PERM), at 3% (FERM 3% and PERM 3%) and 5% (FERM 5% and PERM 5%) concentrations in the culture medium, and 3% (PERM 3%) and 5% (PERM 5%) dilutions in the culture medium, shown with the crystal violet (A) and intracellular LDH activity measurement (B) assays;

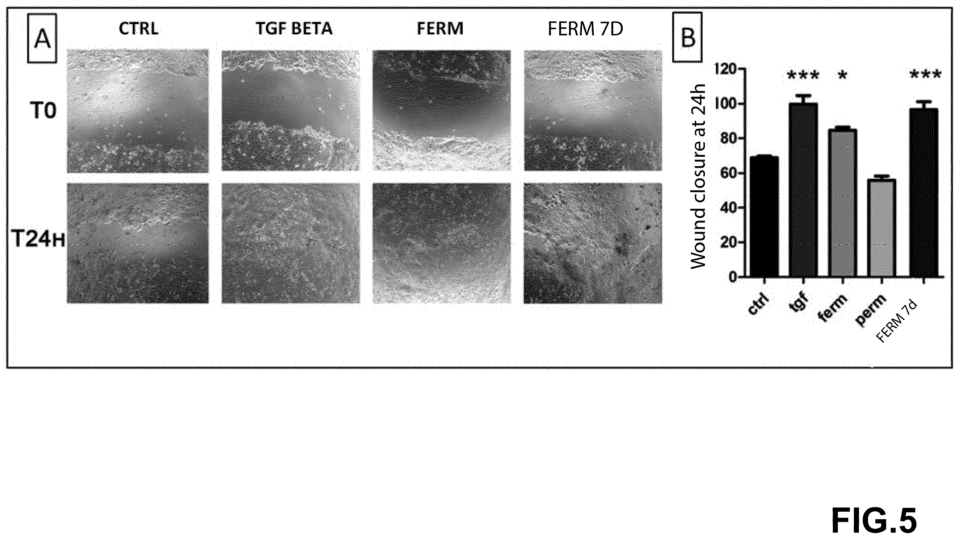

Figure 5 shows the results of the wound healing assay. (A) Micrographs representing a wound at time 0 (T0) and after healing (T24H), in the absence of treatment (CTRL) and after treatment with the composition of the invention (FERM) for 24 hours (T24H) or for 7 days and then tested for 24 hours (FERMG). (B) A graph showing the percentage of wound healing after the following treatments: "aqueous whey fraction" (perm), composition of the invention (ferm) for 24 hours (T24H) or for 7 days and then tested for 24 hours (FERM 7d). The wound treated with the composition of the invention, under both conditions (ferm and FERM7d), heals more rapidly than the untreated wound (ctrl) and significantly better than the "aqueous whey fraction" (PERM). Treatment with TGF beta represents the positive control of the experiments (wound healing-stimulating treatment);

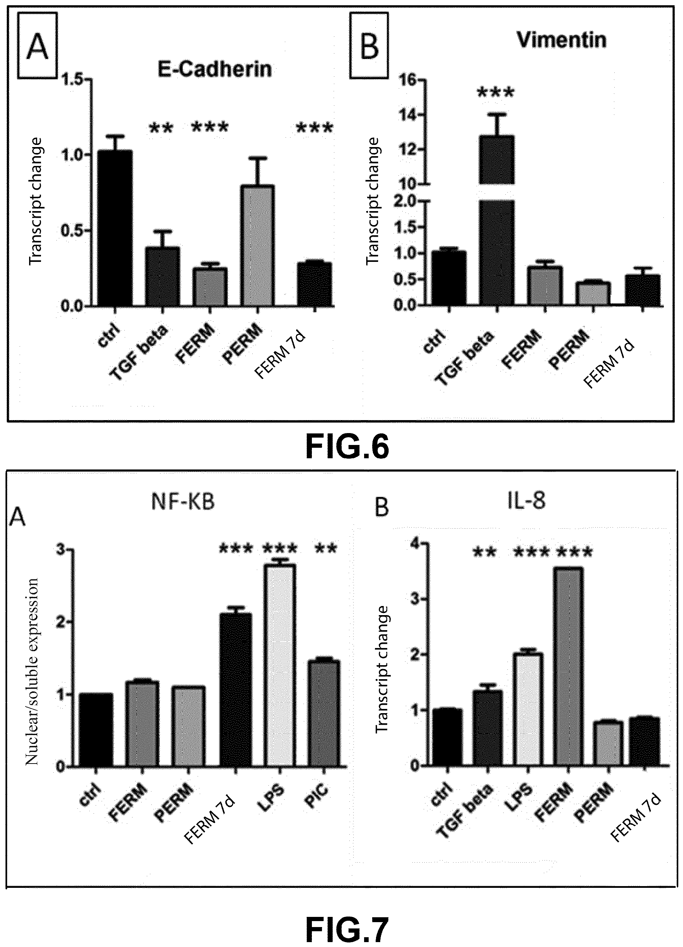

Figure 6 shows graphs representing expression levels in epithelial cells of E-Cadherin (A), a protein required in cell-cell contact, and Vimentin (B), a tumour transforming protein, after treatment with the composition of the invention (FERM and FERM7d) and with the "aqueous whey fraction" (PERM). The composition of the invention (FERM), unlike the "aqueous whey fraction" (PERM), decreases the expression of E-Cadherin (A) whereas it does not induce an increase in Vimentin (B). TGF beta was used as a positive control;
Figure 7 shows graphs representing the induction in epithelial cells of the activity of NF-kB transcription factor (A) and the production of the inflammatory interleukin 8 (IL-8) cytokine (B) by the composition of the invention after 24 hours (FERM) or 7 days of treatment (FERM 7d) and by the aqueous whey fraction (PERM). The data demonstrate the activity of the composition of the invention already after 24 hours of treatment. Cells were also treated with LPS and PIC, i.e., two substances that activate TLR receptors, to show that the effect of the composition of the invention is probably mediated by these receptors. Treatment for 7 days with the fermented permeate (FERM 7 d) no longer induces the inflammatory cytokine IL-8, therefore it is a transient and reversible inflammation that can be considered beneficial;

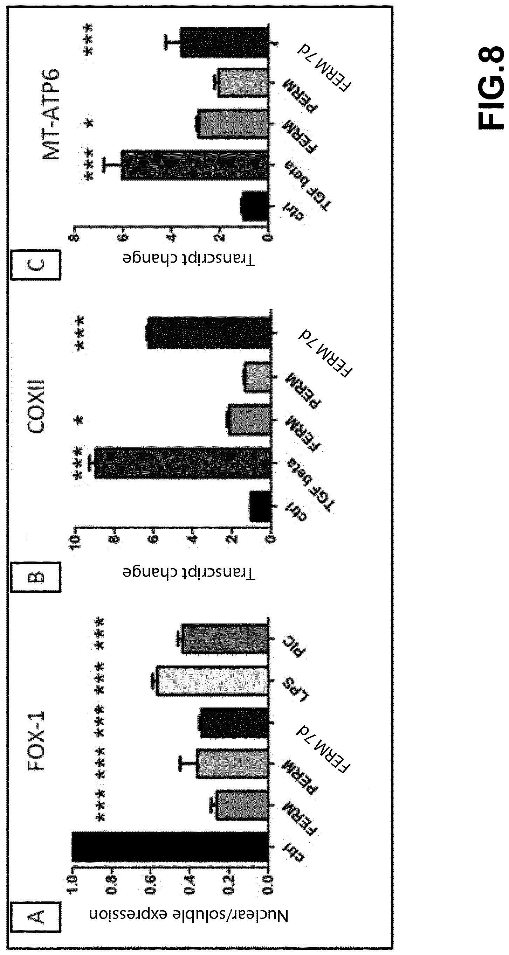

Figure 8 shows graphs depicting cell expression of FOXO1 transcription factor (A) and of the mitochondrial respiratory proteins COXII (B) and MT-ATP6 (C) in the absence of treatment (CTRL) and after the following treatments: composition of the invention for 24 hours (FERM) or for 7 days (FERM 7d); aqueous whey fraction (PERM); LPS and PIC, i.e., two substances that activate TLR receptors; TGF beta. The composition of the invention (FERM), already after 24h of treatment, reduces the presence of FOXO1 transcription factor (A) in the mitochondrion and increases the expression of the mitochondrial respiratory proteins COXII (B) and MT-ATP6 (C). Treatment for 7 days with the preparation (FERM 7 d) amplifies its effects;

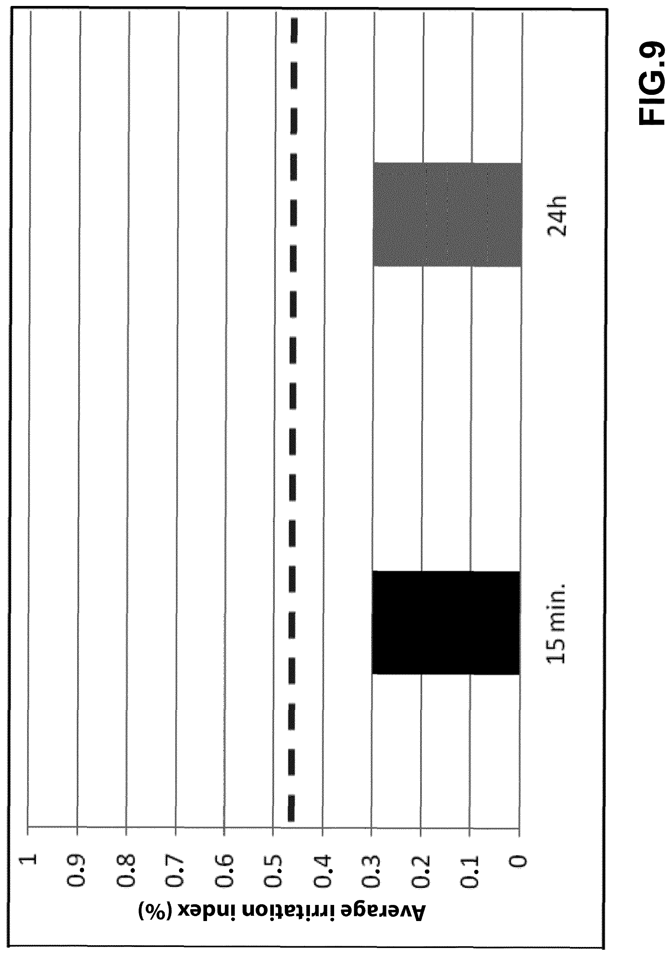

Figure 9 shows a graph depicting the mean index of skin irritation caused after application of the composition of the invention for 15 minutes or 24 hours, compared to the light erythematous response detected. The dotted line indicates the limit beyond which the tested product is slightly irritating.

### Experimental section

The present inventors declare that they have complied with the requirements laid down in the field of biotechnological inventions pursuant to article 170 bis, paragraphs 2, 3 and 4 of Legislative Decree no. 30/05 (Industrial Property Code).

### Example 1: Microbiological characterization of the isolated MF-20/7A/24 strain

The isolated strain *of Lactobacillus delbrueckii* object of the invention belongs to the collection of lactic bacteria of the Institut Agricole Regional (IAR; Regione La Rochère 1/A, Aosta, Italy) selected from mountain pastures and small dairy farms of Valle d'Aosta producers who process milk following the ancient family tradition and without the use of commercially available enzymes, in the production area of Fontina DOP cheese.

The microbiological characterization of the lactobacillus of the invention was carried out by assessing the growth temperature, the medium and the growth conditions, the antibiotic resistance by means of an antibiogram, the bacterial morphology by means of microscopic analysis, the ability to produce the catalase enzyme by means of the catalase test, and to ferment lactose, glucose, and galactose by means of a specific test on a selective medium (Liofilchem srl, Italy). The natural ability to ferment galactose is unusual for *Lactobacillus delbrueckii,* since it has been reported in literature only for Lactococci and *Lactobacillus helveticus.* In addition, β-galactosidase (ONPG) activity was assessed and confirmed by rapid Remel RapID^{™} ONE System test (Thermofisher Scientific, Italy).

### Example 2: Genetic characterization of the isolated MF-20/7A/24 strain

The present inventors carried out a phylogenetic study of the novel isolated strain of *Lactobacillus delbrueckii* by Multi Locus Sequence Tagging (MLST) analysis using 14 native Valle d'Aosta *L*. *delbrueckii* isolates (IAR collection; Regione La Rochère 1/A, Aosta, Italy) from milk being processed for the production of Fontina cheese and three non-native commercially available enzymes (Lyofast, Sacco S.r.l., Cadorago, Como, Italy) commonly used for the production of yoghurt.

Bacterial genomic DNA extraction was performed using the DNeasy Blood and Tissue Kit (QIAGEN), following the procedure for Gram-positive bacteria. All DNA samples after extraction were subjected to MLST analysis, using the primers shown in Table 1, by PCR amplification and sequencing of nine loci suitable for the identification and characterization of lactic bacteria (Shehata et al. Annals of Agricultural Science (2016)61: 65-75; Song et al. Scientific Reports (2016) 6: 22704).

**Table 2**

| Primer name | Sequence (5'-3') | Target locus | Bibliographic reference |
|---|---|---|---|
| Forward LAB-F | CGTGCCAGCCGCGGTAATA CG (SEQ ID NO. 10) | 16S rDNA | Shehata et al. (2016) |
| Reverse LAB-R | GGGTTGCGCTCGTTGCGGG ACTTAACCCAACAT (SEQ ID NO. 11) | 16S rDNA | |
| Forward clpX_F | AAGTCTGCCAGCCAAGTAA (SEQ ID NO. 12) | ATP-dependent Clp protease | Song et al. (2016) |
| Reverse clpX_R | CGCCGACAATGAAGAGGA T (SEQ ID NO. 13) | ATP-binding subunit ClpX (ClpX) | |
| Forward dnaA_F | GAGCCCAGCAGCGAAAG (SEQ ID NO. 14) | Chromosomal replication initiation protein DnaA | |
| Reverse dnaA_R | CTAAGCCAATGTTGATGTC C (SEQ ID NO. 15) | | |
| Forward groEL_F | TCGGCAAGGACGGTGTT (SEQ ID NO. 16) | GroEL chaperone protein | |
| Reverse groEL_R | GTGGATTACGGCTACGC (SEQ ID NO. 17) | | |
| Forward murE_F | CTGAGCAGCCATACCCG (SEQ ID NO. 18) | UDP-N-acetylmuramoyl-L-alanyl-D-glutamate-L-lysine ligase | |
| Reverse murE_R | CCTTGCTTGAAGCCGTAG (SEQ ID NO. 19) | | |
| Forward pheS_F | CATCGGCATGAGCTACCA (SEQ ID NO. 20) | Phenylalanine-tRNA ligase alpha subunit | |
| Reverse pheS_R | CCTCCTGACGGAATTGTTG (SEQ ID NO. 21) | | |
| Forward pyrG_F | AAGCCGACCCAGCAATC (SEQ ID NO. 22) | CTP synthetase | |
| Reverse pyrG R | AGCCCAGACGCAAGGTG (SEQ ID NO. 23) | | |
| Forward recA_F | ATGCGGATGGGCGAGAA (SEQ ID NO. 24) | Recombinase A | |
| Reverse recA_R | CT ACCTT AAA TGGCGGAGC (SEQ ID NO. 25) | | |
| Forward rpoB_F | GGCGGAAAGAGTTATCGT (SEQ ID NO. 26) | DNA-dependent RNA polymerase beta subunit | |
| Reverse rpoB_R | GATGTCGGCTGGAGTGAT (SEQ ID NO. 27) | | |

PCR analyses were carried out using the VERITI thermal cycler (Thermo Fisher Scientific) and following the parameters and conditions indicated in *ad hoc* prepared operating sheets. The amplified DNA fragments were subjected to agarose gel electrophoresis run, visualized by UV transilluminator and quantified by GelAnalyzer software v. 2010a after comparison with DNA fragments of known length and concentration (100bp DNA Ladder, Promega). The amplified samples were then subjected to enzymatic purification by Exosap-IT kit (Affymetrix Inc.) to remove excess primers and nucleotides.

The purified samples were then subjected to Cycle sequencing reaction using the BigDye^{™} Terminator v.3.1 Cycle Sequencing kit (Thermo Fisher Scientific). The Cycle sequencing products were purified to remove excess of unincorporated labelled dideoxynucleoside triphosphates, which could cause problems in subsequent sequence reading. Purification was performed by ethanol precipitation reaction using EDTA as a stabilizer for the labelled DNA fragments. The purified products were then resuspended in the Hi-Di^{™} Formammide solution (Thermo Fisher Scientific) and denatured. The samples were then loaded onto the AB3500 Genetic Analyzer Sequencer (Thermo Fisher Scientific) and subjected to capillary electrophoresis.

The sequences obtained were analysed by SeqA 6 software (Thermo Fisher Scientific), which allows selection of portions with a high percentage of bases having Quality Value (QV)>20 (i.e., probability that that base is randomly selected <1%).

The clean sequences were then analysed with the BioEdit software v.7.2.5 which allows the forward and reverse sequences of each sample and the corresponding electropherograms to be compared, by aligning them with the ClustalW function, for further sequence quality control and to obtain, thanks to their combination, a consensus sequence. This sequence was then saved as a text file in the FASTA format.

In order to assess or confirm the species to which they belong and to carry out a further control, all the sequences obtained were analysed by BLASTn in GenBank (www.blast.ncbi.nlm.nih.gov/Blast.cgi).

For each locus, the sequences obtained from each of the two species were aligned by MEGA 7 software v.7.0.26 to reveal any intra-species polymorphisms and identify the haplotypes therein. The sequences of each locus were linked and aligned with other co-specific sequences available in GenBank. Distance matrices have thus been calculated, which made it possible to obtain dendrograms useful for visually assessing the detected haplotypes and their relative phylogenetic distance.

Analysis of the nine sequenced loci showed three different haplotypes, of which one is characteristic of the MF-20/7A/24 isolate object of the invention (haplotype II), one is common to all the other native Valle d'Aosta isolates (haplotype I), and one is common to commercially available strains (haplotype III).

A phylogenetic tree was created (Figure 1) by aligning the linked sequences of the different haplotypes (for a total of 5,362 bases compared) with those of other strains belonging to the different *L. delbrueckii* subspecies available in GenBank, which revealed the uniqueness of the haplotype II corresponding to the MF-20/7A/24 isolate and its phylogenetic proximity to an *L. delbrueckii* ssp. indicus strain (GenBank accession no. CP018614). Phylogenetic analysis also showed the uniqueness of the haplotype I common to the other native Valle d'Aosta isolates and its phylogenetic proximity to a strain of *L. delbrueckii* ssp. lactis (GenBank accession no. CP018215) and *L. delbrueckii* ssp. delbrueckii (GenBank accession no. CP018615), and also that the haplotype III, comprising the commercially available strains, fits perfectly in the group that contains all strains belonging to the subspecies *bulgaricus.*

### Example 3: Expression analysis of the isolated MF-20/7A/24 strain genes involved in the metabolism of galactose and butyric acid

The present inventors carried out a transcriptomic study of the novel isolated strain of *Lactobacillus delbrueckii* by RNA-Seq analysis, thereby identifying differentially expressed orthologous genes compared to a commercially available non-native *Lactobacillus delbrueckii* (6Y450 strain).

The transcriptomic study included a first DNA-Seq analysis step for whole genome sequencing of the MF-20/7A/24 isolate and the 6Y450 strain, and a second RNA-Seq step that used the genomes thus obtained for mapping the generated reads.

The DNA-Seq analysis therefore involved sample preparation and construction of genomic DNA libraries by KAPA Library Preparation Kit and MiSeq Illumina platform with v3 (600 cycles) 2x300bp paired end chemistry sequencing of the libraries with a coverage of at least 20X for each of the species analysed.

Genomes were assembled using A5-miseq (Coil D, Jospin G, Darling AE. A5-miseq: an updated pipeline to assemble microbial genomes from Illumina MiSeq data. Bioinformatics. 2015;31(4):587-589. doi:10.1093/bioinformatics/btu661), a pipeline specifically designed for assembling bacterial genomes sequenced with MiSeq Illumina technology.

The assembled genomes were annotated by using the Prokka software for the annotation of bacterial genomes. The quality of the assemblies obtained was assessed by using the CheckM software which estimates genome completeness and contamination based on universal single-copy marker collections.

RNA-Seq analysis provided enrichment of primary products by rRNA depletion, construction of libraries of transcripts by Kapa Stranded Mma-Seq Kits, and sequencing by MiSeq Illumina platform with v3 (150 cycles) 2x75 bp paired end chemistry sequencing of the libraries with a coverage of about 30X for each of the species analysed. The quality of the collected samples was assessed by using the FastQC software (http://www.bioinformatics.babraham.ac.uk/projects/fastqc/). Samples were pre-processed for adapter removal by using Trimmomatic (version 0.36) and ribosomal sequences were removed by using SortMeRNA. Pre-processed sequences were aligned with individual genomes by using Bowtie2 (version 2.3.4.1). The counts of the pre-processed sequences were extracted from the alignment by using featureCount read summarization program.

Orthologous genes for the comparison of interest between the new isolated strain *of Lactobacillus delbrueckii* and the non-native commercially available strain were identified based on the analysis of the RNAseq data alignment, thereby determining differentially expressed genes, by using limma/voom and comparing the counts of the mapping of the individual samples on the respective genomes.

The results of this test showed that 5 genes involved in galactose metabolism and 7 genes involved in butyric acid metabolism are present and expressed in the genome of the MF-20/7A/24 isolate and that all these genes are clearly over-expressed compared to orthologous genes identified in the commercially available strain used as a comparison (Table 3). Moreover, orthologous genes were not found for three genes (galT, gabD1 and th1A) in the commercially available strain (Table 3).

**Table 3**

| **Gene** | **Enzyme** | **Pathway** | **Counts MF-20/7A/24 *** | **Counts 6Y450*** |
|---|---|---|---|---|
| lacZ (SEQ ID NO. 28) | Beta-galactosidase | Galactose metabolism | 5588 | 146 |
| galT (SEQ ID NO. 29) | Galactose 1-phosphate uridylyltransferase | | 803 | - |
| galK_1 (SEQ ID NO. 30) | Galactokinase | | 572 | 3 |
| glf (SEQ ID NO. 31) | UDP-galactopyranose mutase | | 233 | 2 |
| galE (SEQ ID NO. 32) | UDP-glucose 4-epimerase | | 1413 | 26 |
| pyk (SEQ ID NO. 33) | Pyruvate kinase | Butyric acid metabolism | 16955 | 196 |
| ppc (SEQ ID NO. 34) | Phosphoenolpyruvate carboxylase | | 5056 | 21 |
| pox5_1 (SEQ ID NO. 35) | Pyruvate oxidase | | 3086 | 5 |
| pox5_2 (SEQ ID NO. 36) | Pyruvate oxidase | | 848 | 1 |
| ackA (SEQ ID NO. 37) | Acetate kinase | | 810 | 6 |
| gabD1 (SEQ ID NO. 38) | Succinate-semialdehyde dehydrogenase [NADP(+)] 1 | | 514 | |
| thlA (SEQ ID NO. 39) | Acetyl-CoA acetyltransferase | | 70 | |
| ^{∗} Number of reads mapped to the genome at corresponding genes | | | | |

The new isolated strain *of Lactobacillus delbrueckii* (designated herein as MF-20/7A/24) was deposited on March 12, 2020, at the Belgian Coordinated Collections of Microorganisms (BCCM) Laboratorium voor Microbiologie - Bacteriënverzameling (LMG) International Depository Authority, Universiteit Gent, K.L. Ledeganckstraat 35, 9000 Gent, Belgium, under accession number LMG P-31789.

### Example 4: Method for the preparation of the dermatological composition of the invention

The present inventors set up a preparation protocol following the method of Fauquant et al. 1985, (Fauquant J. et al, Le Lait (1985), 65(1), 1-20) as modified by Pereira C.D. et al., (Pereira C.D. et al., International Dairy Journal, 2002 12(9): 773-783).

### Whey clarification

In order to remove the lipid component from a sweet dairy whey, the inventors employed a method of thermocalcic precipitation of lipids that can not be centrifuged. More specifically, the Ca²⁺ value of the sweet dairy whey was increased to 1.2 g/l by adding CaCh, then the pH value of said whey was raised to a value in the range of 7.3 to 7.5 with 10N NaOH, quickly followed by hot incubation at the temperature of 55°C for 30 minutes. At the end of the heat treatment, the sweet dairy whey was allowed to cool at room temperature and subsequently was left at 4-6°C for 72 hours, avoiding movements that could disturb the formation of the precipitate. After this time, the supernatant was taken and subjected to centrifugation at 1600 x g at 4°C for 15 minutes; this operation was repeated twice, taking the lower part each time.

The dairy whey thus clarified was subjected to ultrafiltration.

### Whey ultrafiltration

The present inventors performed an ultrafiltration step in order to remove the protein component from the sweet dairy whey.

A Cogent µScale tangential flow filtration device (Merck Millipore) with Pellicon XL polyethersulfone (PES) cassette, with a surface of 0.005m2 and a cut-off of 10 kDa, was used for the ultrafiltration method. The flow rate was fixed at 25 ml/min, with a transmembrane pressure of 20 psi and a temperature ranging from 23.6 to 24.4°C. The portion of whey that passed through the membrane (permeate) was collected and used for the subsequent steps.

### Permeate fermentation

The fermentation protocol provided the use of the *Lactobacillus delbruekii* strain isolated for the first time by the present inventors, designated herein as MF-20/7A/24 and deposited on March 12, 2020 at the International Depository Authority BCCM/LMG (University of Gent, Belgium), with accession number LMG P-31789.

The *Lactobacillus delbruekii* strain obj ect of the invention was used in the freeze-dried form, inoculated in MRS broth culture medium, and incubated at 37°C for 24 hours. The bacterial culture thus obtained was diluted 1:100 and used to ferment the aqueous fraction of the sweet dairy whey (whey permeate) obtained as a result of the thermocalcic precipitation and ultrafiltration steps carried out as described above. Said aqueous fraction of the sweet dairy whey was previously filtered and pasteurized in an autoclave at a temperature of 90°C for 20 minutes.

The fermentation step of the method according to the invention was carried out for 24 hours at 37°C, at the end of which, in order to stop the process, the permeate was placed in an autoclave at 100°C for 15 minutes. Finally, the fermentate was subjected to microfiltration (0.22 µm) to remove the microbial residue.

### Example 5: Characterization of the bioactive components in the dermatological composition of the invention

The present inventors tested the dermatological composition of the invention in order to determine and confirm the presence of potentially bioactive components. It is known from the scientific literature that sweet dairy whey mainly consists of water, mineral salts, sugars (simple and complex), and amino acid residues with a molecular weight of less than 10 kDa.

For the characterization, the non-fat solid components (MSNF, milk solids non-fat) were initially quantified using the official AOAC protocols for milk, adapted to the whey matrix:
- total solids were quantified by the AOAC 990.20 method;
- amino acid residue analysis was carried out by the official Kjeldhal AOAC 991.21 method for non-protein nitrogen;
- mineral salts were quantified by the official AOAC 945.546 method;
- Megazyme kits specific for lactose, galactose, and glucose (codes K-LOLAC, K-ARGA, GLUHK) were used for the glucose components.

From the difference between total solids and all the components listed above, it was possible to indirectly detect and confirm the presence of galacto-oligosaccharides (GOS) in the sample.

Finally, the volatile fraction was qualitatively and quantitatively tested in order to determine and confirm the presence of further bioactive components such as, for example, butyric acid, a saturated fatty acid with 4 carbon atoms. The above step involved the use of an SPME-GC/MS chromatographic method using an 85 µm SPME Carboxen/PDMS StableFlexTM fiber and a DB-Waxcolumn (30 m x 0.25 mm x 0.25 µm film thickness).

The analysis described above showed that, while 0.00153 g/l of butyric acid (0.52 µM) are found in the aqueous whey fraction after ultrafiltration (permeate), a value equal to 0.00514 g/l (1.75 µM) of this organic acid is reached in the dermatological composition of the invention, and this increase is statistically significant (p<0.05) (Figure 2).

### Example 6: In vitro assays in cell models

In order to analyse the properties of the dermatological composition according to the invention and its effects on the skin, the present inventors tested the characteristics of said composition and of the sweet dairy whey aqueous fraction obtained after thermocalcic precipitation and ultrafiltration (hereinafter referred to as the "aqueous whey fraction") in commercially available HaCat human keratinocyte cell lines (American Type Culture Collection (ATCC) (USA)). This cell line originates from adult skin and maintains full epidermal differentiation ability, therefore representing skin tissue. *In vitro* assays suitable for assessing the toxicity and biological activity of the dermatological composition according to the invention were performed on all samples.

### Toxicity assessment

In order to assess the possible toxicity of the dermatological composition according to the invention, the MTT, Neutral Red uptake (NRU), Crystal Violet and LDH release assays were performed, as shown in Table 4 below. Suitable dilutions of the composition of the invention and of the aqueous whey fraction corresponding to 3% or 5% final concentration in the cell growth medium were used in the assays. The sodium dodecyl sulfate (SDS) compound was used as a toxicity positive control (Figure 3).

**Table 4**

| TOXICITY ASSESSMENT | |
|---|---|
| Assay | Purpose of the analysis |
| CRYSTAL VIOLET | Assessing the potential to inhibit skin and mucous physiological growth using the water-soluble CV dye with nuclear affinity: the staining, as measured spectrophotometrically, is proportional to the number of cells |
| LDH LEAKAGE | Colorimetric assay for mortality and cell lysis quantitation, based on the enzymatic activity of LDH (lactate dehydrogenase) released from the cytosol to the supernatant by the damaged cell |
| INTRACELLULAR LDH ACTIVITY | The enzymatic activity considered represents an index of cellular energy metabolism (glycolysis and oxidative phosphorylation). This assay assesses any disruptions due to the presence of the tested product |
| MTT | Cell survival test that uses the action of mitochondrial dehydrogenase enzymes of viable cells to cleave the MTT molecule into formazan (yellow □ purplish), which is spectrophotometrically detectable. An increase or decrease in viable cells results in a concomitant change in the amount of formazan that is formed and can be considered as an indicator of the degree of cytotoxicity caused by exposure to the test substance |
| NEUTRAL RED UPTAKE | Potential skin irritation measured based on the ability of viable cells to bind NR, a positively charged dye that easily diffuses |
| | across cell membranes and, by accumulating in the cytosol, is incorporated into the acidic environment of lysosomes. Only cells with an intact plasma membrane will be able to retain NR |

Cell assays carried out as described above showed that the dermatological composition of the invention and the aqueous whey fraction are not cytotoxic in the short or long term, and do not interfere with the growth and cytoplasmic energy metabolism of human keratinocytes (Figure 4).

### Assessment of the biological activity

In order to examine the biological activity of the dermatological composition according to the invention, the wound healing test, using TGF β as a positive control, and gene and protein expression assays were carried out, as shown in Table 5 below.

**Table 5**

| ASSESSMENT OF THE BIOLOGICAL ACTIVITY | |
|---|---|
| Assay | Purpose of the analysis |
| WOUND HEALING | After the creation of a wound (empty space) on a monolayer of confluent cells, the ability of the cells to move, thereby closing the slit created, is measured: photographs are taken from time 0 to the next 24 hours in order to calculate the wound healing percentage by means of software |
| GENE EXPRESSION | Transcriptional analysis (RT-qPCR) to investigate the molecular mechanisms responsible for wound healing: |
| | • Energy production (amplification of the COX II and MT-ATP6 protein subunit transcripts, derived from mitochondrial DNA, is an indication of the expression of the mitochondrial proteins involved in the production of energy necessary for movement) |
| | • Changes in the transcription of genes involved in cell adhesion (E-cadherin is the protein responsible for cell-cell junctions in the |
| | epithelium, whereas Vimentin is a typical protein of undifferentiated cells) |
| | • Production of inflammatory cytokines that have a positive effect on wound healing (interleukins IL-6, IL-8, IL-24, and TNF-α) |
| | • Transcription of cell differentiation marker genes (involucrin, keratin 1, and keratin 10) |
| PROTEIN EXPRESSION | After separating the cytosol, mitochondrial and nucleus fractions, the western blotting technique is used to analyse the molecular signalling pathway that mediates the effect of the fermented permeate. Specifically, the translocation of the transcriptional factor NFkB from the cytosol to the nucleus and of the transcriptional factor FOXO-1 from the mitochondrion to the nucleus is assessed; in the nucleus they induce IL-8 production (promotion of inflammatory response) and an increase in mitochondrial respiration, respectively |

In the assays, the composition of the invention and the aqueous whey fraction were used at the concentrations described above with reference to the toxicity assessment.

As shown in Figure 5, the wound healing test results unequivocally indicate that the dermatological composition according to the invention exerts a healing effect by promoting wound closure, and that this effect is markedly higher than the aqueous whey fraction.

With the gene expression studies herein, the present inventors investigated the action exerted by the dermatological composition according to the invention on the molecular mechanisms underlying wound closure. In particular, when a wound heals, the epithelial cells move and participate in the inflammatory stimulus.

Gene transcription analysis experiments revealed a significant increase in cell transcription of mitochondrial genes encoding energy production and oxidative phosphorylation proteins by the dermatological composition according to the invention. This can be explained by the cells' need for more energy in order to move. Moreover, the above experiments have shown in the cells treated with the dermatological composition of the invention a significant reduction in the transcriptional levels of E-Cadherin, the protein responsible for cell adhesion in the epithelium (Figure 6A). As a result, the cells are freer to move. Importantly, the above-described transcriptional changes were not found in cells contacted with the aqueous whey fraction alone (Figure 6A).

The gene expression assay also assessed, as a positive control, the effect on the cells of TGF-β, a cytokine that modifies the characteristics of the epithelium and promotes loss of differentiation (epithelial-mesenchymal transition). As can be seen from the graphs in Figure 6, the TGF-β factor in fact reduces E-Cadherin expression but induces the transcription of the gene encoding the Vimentin protein.

In contrast, the dermatological composition and the aqueous whey fraction do not affect the transcription of Vimentin (Figure 6B), and therefore their effect is not mediated by this type of transition which is typical of tumour transformation. This is very important since it implies that the effect of the dermatological composition object of the invention is to increase cell movement without modifying epithelial differentiation.

Treatment of epithelial cells with the dermatological composition of the invention also stimulates the expression of interleukin 8 (IL-8), a cytokine with beneficial effects on wound closure (Figure 7B), and of proteins involved in keratinocyte differentiation, thereby favouring the formation and maintenance of an intact and functional epidermal barrier.

The results of the gene expression studies described above were then also confirmed by the present inventors at the protein expression level. In particular, following treatment of keratinocytes with the dermatological composition of the invention, activation of the transcription factor NF-kb, responsible for the production of IL-8 (Figure 7A), resulting in promotion of the inflammatory response, and migration of FOXO-1 from the mitochondrion, where it is located to repress respiration, to the nucleus (Figure 8), resulting in an increase in mitochondrial respiration, were shown.

### Assessment of the irritating power in subjects with sensitive skin

The objective of the study conducted by the present inventors was to assess the irritating power of the dermatological composition according to the invention in subjects (adult volunteers of both sexes) with sensitive skin, by means of delayed-reading epicutaneous tests (patch tests).

Sensitive, reactive, or hyper-reactive skin is defined as a skin subject to unpleasant sensations such as pinching, burning, pain, itching, or tingling in response to stimuli which should not normally cause such sensations, for example UV rays, hot and dry air, cold and humid air, wind, smog/pollutants, contact with water, use of soaps or cosmetics, psychic stress. We cannot speak of sensitive skin when these unpleasant sensations are attributable to the presence of a skin disease.

In addition to unpleasant sensations, the skin may appear erythematous, although it often maintains a normal appearance. Sensitive skin may be present in any part of the body but is most frequent on the face.

The selection of volunteers for the study was conducted using two combined approaches:
- Self-assessment test (Misery modified);
- Lactic acid test or Ramette test (stinging test).

The lactic acid test or Ramette test consists in applying a 15% lactic acid aqueous solution in the cheekbone region with cotton wool, and distilled water in the contralateral site, as a control. The appearance of stinging indicates the presence of subjective irritation, even in the absence of any type of visible skin reaction. 2 and 5 minutes after application of lactic acid, the test subject will assign a score from 0 to 3 to the burning based on the intensity, as follows:
0 = absence of abnormal sensations;
1 = light burning-heat sensation;
2 = moderate burning-heat sensation;
3 = intense burning-heat sensation.

Volunteers with a cumulative score (after 2 and 5 minutes) ≥3 are considered as *"STINGERS".*

Subjects who consider themselves as affected by sensitive skin by answering yes to the question "do you think your skin is sensitive?" and who exhibit a cumulative score of ≥3 based on the self-assessment questionnaire and a score of ≥3 based on the lactic acid test were diagnosed with sensitive/hyper-reactive skin.

The dermatological composition according to the invention was tested in a single dose (10 µl) on 20 volunteers. Samples were placed in aluminium Finn chambers (Bracco), which were applied to the skin of the volar surface of the forearm with a porous patch, protected by Fixomul hypoallergenic adhesive tape.

The patches with the sample were left *in situ* for 48 hours, reminding the patient not to wash/wet the area throughout the test.

The removal was carried out by the investigator, who cleans the application areas of any product residues. The results are read 15 minutes and 24 hours after the removal of the products, i.e., 48 and 72 hours after their application.

The results were analysed by using the following rating scale for irritant contact dermatitis:
Absent → 0
Mild → 1
Moderate→ 2
Severe → 3

The irritation indices obtained in the 20 tests were averaged.

The product is then classified as follows:
Average irritation index: <0.5 NON-irritant;
Average irritation index: 0.5-20 SLIGHTLY irritant;
Average irritation index: 20-50 MODERATELY irritant;
Average irritation index: 50-80 HIGHLY irritant.

The studies carried out as previously illustrated showed that the dermatological composition according to the invention is not irritant and is also suitable for use on sensitive skin (Figure 9

## Claims

1. An isolated strain of *Lactobacillus delbruekii,* **characterised in that** it comprises:
(a) a genomic nucleic acid sequence encoding 16S ribosomal RNA comprising the nucleotide sequence of SEQ ID NO. 1; and
(b) a genomic nucleic acid sequence selected from the group consisting of a sequence encoding the ATP-dependent Clp protease ATP-binding subunit ClpX (ClpX) comprising the nucleotide sequence of SEQ ID NO. 2, a sequence encoding the chromosomal replication initiation protein DnaA comprising the nucleotide sequence of SEQ ID NO. 3, a sequence encoding the GroEL protein comprising the nucleotide sequence of SEQ ID NO. 4, a sequence encoding the UDP-N-acetylmuramoyl-L-alanyl-D-glutamate-L-lysine ligase protein (MurE) comprising the nucleotide sequence of SEQ ID NO. 5, a sequence encoding the phenylalanine-tRNA ligase alpha subunit (PheS) comprising the nucleotide sequence of SEQ ID NO. 6, a sequence encoding the CTP synthetase (PyrG) comprising the nucleotide sequence of SEQ ID NO. 7, a sequence encoding recombinase A (RecA) comprising the nucleotide sequence of SEQ ID NO. 8, a sequence encoding the DNA-dependent RNA polymerase beta subunit (RpoB) comprising the nucleotide sequence of SEQ ID NO. 9, a sequence encoding the beta-galactosidase protein comprising the nucleotide sequence of SEQ ID NO. 28, a sequence encoding the galactose-1-phosphate uridylyltransferase protein (GalT) comprising the nucleotide sequence of SEQ ID NO. 29, a sequence encoding the galactokinase protein comprising the nucleotide sequence of SEQ ID NO. 30, a sequence encoding the UDP-galactopyranose mutase protein (GIF) comprising the nucleotide sequence of SEQ ID NO. 31, a sequence encoding the UDP-glucose 4-epimerase protein (GalE) comprising the nucleotide sequence of SEQ ID NO. 32, a sequence encoding the pyruvate kinase protein comprising the nucleotide sequence of SEQ ID NO. 33, a sequence encoding the phosphoenolpyruvate carboxylase protein comprising the nucleotide sequence of SEQ ID NO. 34, a sequence encoding the pyruvate oxidase protein (Pox5_1) comprising the nucleotide sequence of SEQ ID NO. 35, a sequence encoding the pyruvate oxidase protein (Pox5_2) comprising the nucleotide sequence of SEQ ID NO. 36, a sequence encoding the acetate kinase protein comprising the nucleotide sequence of SEQ ID NO. 37, a sequence encoding the succinate-semialdehyde dehydrogenase protein [NADP(+)] (GabD) comprising the nucleotide sequence of SEQ ID NO. 38, a sequence encoding the acetyl-CoA acetyltransferase protein comprising the nucleotide sequence of SEQ ID NO. 39, and any combination thereof.

2. The isolated strain of *Lactobacillus delbruekii* according to claim 1, which is deposited at the Belgian Coordinated Collections of Microorganisms (BCCM) Laboratorium voor Microbiologie - Bacteriënverzameling (LMG) International Depository Authority, Gent, Belgium, with accession number LMG P-31789.

3. A method for preparing a dermatological composition for topical application to the skin, employing sweet dairy whey as the starting material, the method comprising the steps of:
(i) removing the lipid and protein components from said whey, thereby obtaining an aqueous fraction of the sweet dairy whey;
(ii) subjecting said aqueous fraction to fermentation by using the isolated strain of *Lactobacillus delbruekii* as defined in claim 1 or 2.

4. The method according to claim 3, wherein the sweet dairy whey is obtained from bovine milk.

5. The method according to claim 3 or 4, wherein the removal of the lipid component from the sweet dairy whey is carried out by thermocalcic precipitation.

6. The method according to any one of claims 3 to 5, wherein the removal of the protein component from the sweet dairy whey is carried out by ultrafiltration.

7. A dermatological composition for topical application to the skin comprising one or more galacto-oligosaccharides and butyric acid, **characterised in that** it is obtainable through the method according to any one of claims 3 to 6.

8. The dermatological composition for topical application to the skin as defined in claim 7, for use in the treatment of a skin disease selected from the group consisting of acne, atopic dermatitis, wounds, burns, and ageing skin damage.

9. Cosmetic use of the dermatological composition as defined in claim 7 for topical application to the skin.
